# FASCICULE DE BREVET EUROPEEN

(11) **EP 0 732 315 B1**
(45) Date de publication et mention de la délivrance du brevet: **18.11.1998**
(21) Numéro de dépôt: 96400324.8
(22) Date de dépôt: 16.02.1996
(51) Int. Cl.: C07C 17/23, C07C 17/395, C07C 19/08

(54) **Procédé d'hydrogénolyse de chlorofluorocarbures et d'hydrochlorofluorocarbures**
Verfahren zur Hydrogenolyse von Chlorfluorkohlenwasserstoffen und Hydrochlorfluorkohlenwasserstoffen
Process for the hydrogenolysis of chlorofluorocarbons and hydrochlorofluorocarbons

(30) Priorité: 17.03.1995 FR 9503117
(43) Date de publication de la demande: 18.09.1996
(73) Titulaire: ELF ATOCHEM S.A., 92800 Puteaux, Hauts-de-Seine (FR)
(72) Inventeur: Guillet, Dominique, 69390 Vernaison (FR); Hub, Serge, 69007 Lyon (FR)
(74) Mandataire: Leboulenger, Jean

(56) Documents cités:
- EP-A- 0 506 525
- WO-A-94/02439
- FR-A- 2 645 531
- US-A- 5 136 113

## Description

La présente invention concerne un procédé d'hydrogénolyse de chlorofluorocarbures (CFC) et d'hydrochlorofluorocarbures (HCFC).

Il semble aujourd'hui clairement établi que les responsables de la diminution de la couche d'ozone dans la stratosphère sont les chlorofluorocarbures. En effet, les CFC libérés tendent à percoler lentement vers la stratosphère où il se décomposent par photodissociation en libérant du chlore monoatomique. L'atome de chlore détruit les molécules O₃ (ozone) au cours de cycles catalytiques où il se régénère, pouvant ainsi affecter plusieurs molécules. La communauté internationale a donc décidé d'abandonner la production de CFC et il a fallu dès lors trouver et produire des substituts acceptables.

Les CFC sont composés, comme leur nom l'indique, d'atomes de chlore, de fluor et de carbone, mais sont dépourvus d'hydrogène. Une stratégie envisagée par les producteurs consiste à leur substituer des molécules comportant les mêmes éléments plus de l'hydrogène, donc moins stables et capables de se dégrader rapidement dans la basse atmosphère. L'objectif définitif consistera à utiliser des composés dépourvus de chlore, les hydrofluorocarbures (HFC), dont l'impact sur l'ozone devrait être nul.

Dans ce contexte, l'hydrogénolyse qui remplace dans une molécule un atome de chlore par un atome d'hydrogène, constitue une réaction particulièrement adaptée au problème posé.

La potentialité de cette réaction est démontrée dans de nombreux brevets. Ainsi, l'hydrogénolyse du chlorodifluorométhane en difluorométhane est décrite dans le brevet EP 0 508 660, et celles des chlorotétrafluoroéthane et dichlorotétrafluoroéthane en tétrafluoroéthane sont mentionnées dans les brevets GB 1 578 933, EP 0 349 115 et US 4 873 381. Ce type de réaction constitue également un bon moyen d'épuration des HFC des CFC éventuellement présents, comme décrit dans la demande de brevet WO 94/02439 dans le cas du pentafluoroéthane.

Cependant, l'inconvénient majeur des procédés d'hydrogénolyse se situe dans la stabilité dans le temps de l'activité catalytique. En effet, dans les conditions réactionnelles souvent sévères nécessaires à la transformation total des réactifs, le catalyseur se désactive avec le temps. Il est donc nécessaire de le remplacer périodiquement avec une charge neuve ou de trouver un moyen efficace de régénération du catalyseur usagé.

Dans cette optique, plusieurs techniques de régénération de catalyseurs d'hydrogénolyse se trouvent décrits dans la littérature. La demande de brevet WO 93/24224 propose une oxydation par l'oxygène ou un agent oxydant du catalyseur usagé. Des traitements au chlore (US 5 057 470) ou au CFC qui peut être le réactif à transformer (US 4 980 324) se révèlent également efficaces. Cependant, ces procédés ne font que réactiver les catalyseurs, qui présentent toujours après le traitement les mêmes inconvénients.

Il a maintenant été trouvé que l'incorporation de soufre dans un catalyseur à base de palladium déposé sur un support lui confère la propriété d'être stable dans les réactions d'hydrogénolyse en phase gazeuse, aussi bien dans les réactions de synthèse de HFC à partir de CFC ou de HCFC, que dans des procédés d'épuration d'impuretés CFC contenues dans les HFC.

Le traitement au soufre d'un catalyseur d'hydrogénation/hydrogénolyse est connu du brevet FR 2 645 531 qui décrit le traitement d'un catalyseur Pd/C par des composés soufrés pour augmenter la sélectivité de l'hydrogénolyse en phase liquide de l'acide dichloracétique (HCl₂C-COOH) en acide monochloracétique (H₂ClC-COOH). Cependant, il était totalement inattendu que le traitement du catalyseur avec un composé du soufre permette de stabiliser son activité dans l'hydrogénolyse de CFC ou de HCFC en phase gazeuse, d'autant que le rapport final de recherches de J.D.PARK et J.R.LACHER publié en 1959, recherches financées par l'Air Force Office of Scientific Research (n°TR5899) et l'Armed Services Technical Informations Agency (n°AD 162198), décrit un traitement du support pour éliminer le soufre avant l'imprégnation du palladium.

L'invention a donc pour objet un procédé d'hydrogénolyse en phase gazeuse de chlorofluorocarbures ou d'hydrochlorofluorocarbures en présence d'un catalyseur à base de palladium déposé sur un support caractérisé en ce que l'on incorpore du soufre au catalyseur.

Dans le catalyseur selon l'invention, le support peut être du charbon, une alumine fluorée ou du fluorure d'aluminium, et le palladium est avantageusement déposé sur ce support à raison de 0,1 à 10 % en poids par rapport au poids total de catalyseur (Pd + support).

La quantité de soufre à incorporer au catalyseur peut aller de 0,75 à 750 mg de soufre par gramme de palladium. Elle est de préférence comprise entre 2 et 100 mg de soufre par gramme de palladium, et plus particulièrement entre 7,5 et 75 mg de soufre par gramme de palladium.

Le soufre peut être incorporé au catalyseur avant et/ou pendant son utilisation. L'incorporation peut être réalisée de différentes façons selon que le composé soufré précurseur est normalement liquide (par exemple SCl₂, S₂Cl₂, CS₂, thiophène, diméthyl sulfure,...) ou gazeux (par exemple H₂S, méthylmercaptan...).

Lorsque le composé soufré précurseur est un liquide, on peut opérer par imprégnation en présence d'un solvant dont le choix dépend de la nature du précurseur soufré. Dans le cas de CS₂, l'éthanol est particulièrement adapté; tout solvant de CS₂ peut cependant être utilisé. Après l'imprégnation, le catalyseur est traité thermiquement sous atmosphère d'un gaz éventuellement inerte, mais on préfère utiliser l'hydrogène, à une température comprise entre 150 et 400°C pour décomposer le composé soufré.

Lorsque le précurseur soufré est normalement gazeux (H₂S, H₃C-SH) ou est un liquide possédant une forte tension de vapeur (par exemple CS₂), il peut être introduit sur le catalyseur par la phase gazeuse avant ou pendant l'admission de l'hydrogène et du réactif à hydrogénolyser. Dans cette technique particulièrement avantageuse de traitement du catalyseur "in situ" dans le réacteur, la quantité de soufre amenée sur le catalyseur peut être ajustée aux teneurs précédemment indiquées en agissant sur la concentration du gaz en composé soufré, le débit du gaz et la durée du traitement.

Quelques soient le composé soufré et son mode d'incorporation, l'introduction de soufre suivie d'un traitement thermique conduit à la formation d'une phase solide de soufre et de palladium de formule Pd₄S. La transformation totale du palladium disponible n'est cependant pas nécessaire pour obtenir un catalyseur stable.

Les conditions opératoires de la réaction d'hydrogénolyse peuvent varier dans de larges limites selon la nature du réactif à hydrogénolyser (CFC ou HCFC) :
- La température de réaction est généralement comprise entre 100 et 450°C, mais on préfère travailler entre 150 et 350°C.
- La pression peut aller de 1 à 50 bars ; une augmentation de pression a pour effet d'augmenter le temps de contact et donc de permettre d'atteindre des conversions élevées pour une température donnée.
- Le débit horaire de réactif alimenté en continu au réacteur peut aller de 0,01 à 12 moles par litre de catalyseur.
- Le rapport molaire H₂/réactif est généralement compris entre 0,5 et 10, de préférence entre 1 et 6.

Comme exemples non limitatifs de réactifs auxquels s'applique le procédé selon l'invention, on peut citer plus particulièrement le chloropentafluoroéthane (F115), le 1,1-dichloro-1,2,2,2-tétrafluoroéthane (F114a), le chlorodifluorométhane (F22), le 1-chloro-1,1-difluoroéthane (F142b) et le 1-chloro-1,2,2,2-tétrafluoroéthane (F124) dont l'hydrogénolyse conduit respectivement au pentafluoroéthane (F125), au 1,1,1,2-tétrafluoroéthane (F134a), au difluorométhane (F32), au 1,1-difluoroéthane (F152a) et au 1,1,1,2-tétrafluoroéthane (F134a). Peuvent également être mentionnés des (hydro)chlorofluorocarbures en C₃ comme le 1,2,2-trichloro-1,1,3,3,3-pentafluoropropane (F215aa) ou le 1,2-dichloro-1,1,3,3,3-pentafluoropropane (F225da) dont l'hydrogénolyse conduit au 1,1,1,3,3-pentafluoropropane (F245fa).

Les exemples suivants illustrent l'invention sans la limiter. Les pourcentages relatifs aux sélectivités sont exprimés en moles.

### EXEMPLE 1 : Comparatif

Dans un réacteur tubulaire en Inconel de 45 cm de long et 2,72 cm de diamètre intérieur, chauffé électriquement, on introduit 75 ml d'un catalyseur Pd/C commercial à 3 % en poids de palladium. Préalablement à l'introduction des réactifs, le catalyseur est réduit à 300°C sous pression atmosphérique d'hydrogène.

On fait passer sur le catalyseur un mélange d'hydrogène, de pentafluoroéthane (F125) et de chloropentafluoroéthane (F115) dans les conditions opératoires suivantes :
- Température: : 330°C
- Débit d'hydrogène: : 0,107 mole/heure
- Débit de F125: : 0,286 mole/heure
- Débit de F115: : 0,018 mole/heure

L'analyse se fait par chromatographie (CPV) en ligne à la sortie du réacteur. Les résultats rassemblés dans le tableau suivant montrent une rapide décroissance de l'activité du catalyseur dans le temps.

| **TEMPS (heures)** | **CONVERSION (%) du F115** | **SELECTIVITE (%) EN** | | |
|---|---|---|---|---|
| | | **F125** | **F134a** | **F143a** |
| 11 | 90,3 | 93,8 | 1,4 | 4,8 |
| 32 | 88,0 | 94,9 | 1,0 | 4,1 |
| 40 | 86,2 | 95,1 | 1,0 | 3,9 |
| 60 | 83,3 | 95,3 | 1,0 | 3,7 |
| 68 | 82,3 | 95,3 | 1,1 | 3,6 |
| 80 | 81,3 | 95,4 | 1,0 | 3,6 |
| 92 | 77,9 | 95,6 | 1,0 | 3,4 |
| 100 | 77,9 | 95,6 | 0,9 | 3,5 |
| 108 | 76,5 | 95,7 | 1,0 | 3,3 |
| 122 | 75,1 | 95,6 | 1,0 | 3,4 |
| 130 | 73,7 | 95,5 | 1,1 | 3,4 |
| 148 | 70,9 | 95,7 | 0,9 | 3,4 |
| 170 | 67,8 | 95,7 | 1,0 | 3,3 |
| 190 | 64,3 | 95,5 | 1,0 | 3,5 |
| 208 | 61,3 | 95,7 | 0,9 | 3,4 |
| 230 | 57,2 | 95,9 | 0,8 | 3,3 |
| 252 | 54,8 | 95,8 | 0,9 | 3,3 |
| 268 | 52,0 | 95,9 | 0,9 | 3,2 |
| 289 | 49,0 | 95,8 | 0,8 | 3,4 |
| 308 | 45,0 | 96,0 | 0,9 | 3,1 |

### EXEMPLE 2

### a) Traitement du catalyseur

Dans un évaporateur rotatif, on charge 75 ml du même catalyseur commercial Pd/C qu'à l'exemple précédent, puis on introduit 100 ml d'une solution d'éthanol contenant 0,011 mole/litre de CS₂. Le solide est maintenu en contact avec la solution à 20°C pendant 20 heures. Le catalyseur est ensuite récupéré par filtration, puis réduit à 300°C sous pression atmosphérique d'hydrogène pendant 4 heures. La quantité de soufre fixé est de 0,2 % en poids et la diffraction des rayons X met en évidence la formation d'une phase Pd₄S.

### b) Purification du F125

Dans le même réacteur tubulaire qu'à l'exemple 1, on introduit 75 ml du catalyseur préparé ci-dessus, puis on fait passer sur ce catalyseur un mélange d'hydrogène, de pentafluoroéthane (F125) et de chloropentafluoroéthane (F115) dans les conditions opératoires suivantes :
- Température: : 330°C
- Débit d'hydrogène: : 0,107 mole/heure
- Débit de F125: : 0,286 mole/heure
- Débit de F115: : 0,018 mole/heure

Les résultats de l'analyse effectuée par chromatographie (CPV) en ligne à la sortie du réacteur sont rassemblés dans le tableau suivant. On constate une stabilité remarquable de l'activité catalytique.

| **TEMPS (heures)** | **CONVERSION (%) du F115** | **SELECTIVITE (%) EN** | | |
|---|---|---|---|---|
| | | **F125** | **F134a** | **F143a** |
| 27 | 59,4 | 76,2 | 6,4 | 17,4 |
| 35 | 60,4 | 76,9 | 6,2 | 16,9 |
| 45 | 60,0 | 81,9 | 6,4 | 11,7 |
| 65 | 63,8 | 84,4 | 5,9 | 9,7 |
| 77 | 61,6 | 84,6 | 6,0 | 9,4 |
| 79 | 63,7 | 84,8 | 5,7 | 9,5 |
| 95 | 60,8 | 84,2 | 6,3 | 9,5 |
| 105 | 63,0 | 85,2 | 5,6 | 9,2 |
| 115 | 62,7 | 84,3 | 5,9 | 9,8 |
| 125 | 62,7 | 84,8 | 5,6 | 9,6 |
| 143 | 62,5 | 84,7 | 5,6 | 9,7 |
| 157 | 62,8 | 83,0 | 5,4 | 11,6 |
| 164 | 65,9 | 86,7 | 5,1 | 8,2 |
| 197 | 65,6 | 86,7 | 5,1 | 8,2 |
| 208 | 62,8 | 87,5 | 5,2 | 7,3 |
| 212 | 61,4 | 85,8 | 5,5 | 8,7 |
| 234 | 62,6 | 85,6 | 5,1 | 9,3 |
| 254 | 62,0 | 85,9 | 5,1 | 9,0 |
| 265 | 62,7 | 86,7 | 5,2 | 8,1 |
| 292 | 63,1 | 87,6 | 5,1 | 7,3 |
| 296 | 63,5 | 87,5 | 4,9 | 7,6 |
| 313 | 63,5 | 88,5 | 5,0 | 6,5 |

### EXEMPLE 3

### a) Traitement du catalyseur

On opère comme à l'exemple 2-a, mais avec 100 ml d'une solution d'éthanol contenant 0,001 mole/litre de CS₂. La diffraction des rayons X ne détecte pas de phase cristallisée Pd₄S, mais l'analyse du soufre montre la présence de 500 ppm en poids de soufre sur le catalyseur.

### b) Purification du F125

Avec le catalyseur ainsi traité et en opérant comme à l'exemple 2-b, on a obtenu les résultats rassemblés dans le tableau suivant :

| **TEMPS (heures)** | **CONVERSION (%) du F115** | **SELECTIVITE (%) EN** | | |
|---|---|---|---|---|
| | | **F125** | **F134a** | **F143a** |
| 21 | 65,8 | 86,7 | 4,9 | 8,4 |
| 46 | 62,6 | 87,6 | 4,6 | 7,8 |
| 70 | 62,7 | 88,5 | 4,6 | 6,9 |
| 80 | 62,8 | 88,7 | 4,4 | 6,9 |
| 96 | 61,0 | 88,2 | 4,5 | 7,3 |
| 101 | 61,6 | 89,2 | 4,3 | 6,5 |
| 115 | 58,9 | 88,6 | 4,5 | 6,9 |
| 127 | 59,9 | 88,8 | 4,0 | 7,2 |
| 176 | 61,2 | 89,4 | 3,8 | 6,8 |
| 185 | 62,6 | 90,6 | 3,5 | 5,9 |
| 195 | 61,9 | 90,2 | 3,4 | 6,4 |
| 205 | 62,0 | 90,8 | 3,5 | 5,7 |
| 215 | 61,0 | 90,2 | 3,5 | 6,3 |
| 243 | 61,6 | 91,1 | 3,1 | 5,8 |
| 251 | 61,6 | 90,7 | 3,1 | 6,2 |
| 273 | 58,4 | 90,5 | 3,3 | 6,2 |
| 315 | 58,7 | 90,7 | 3,1 | 6,2 |
| 325 | 61,0 | 91,0 | 3,0 | 6,0 |

Le niveau de conversion est le même qu'à l'exemple 2-a avec la même stabilité dans le temps. On note en outre une amélioration de la sélectivité en F125.

### EXEMPLE 4 : Comparatif

Dans un réacteur tubulaire en Inconel de 45 cm de long et 2,72 cm de diamètre intérieur, chauffé électriquement, on introduit 75 ml d'un catalyseur Pd/C commercial à 2 % en poids de palladium. Préalablement à l'introduction des réactifs, le catalyseur est réduit à 300°C sous pression atmosphérique d'hydrogène.

On fait passer sur le catalyseur un mélange d'hydrogène, de pentafluoroéthane (F125) et de chloropentafluoroéthane (F115) dans les conditions opératoires suivantes :
- Température: : 250°C
- Débit d'hydrogène: : 0,103 mole/heure
- Débit de F125: : 0,281 mole/heure
- Débit de F115: : 0,018 mole/heure

L'analyse faite par chromatographie (CPV) en ligne à la sortie du réacteur donne les résultats rassemblés dans le tableau suivant. On note une décroissance de l'activité du catalyseur dans le temps.

| **TEMPS (heures)** | **CONVERSION (%) du F115** | **SELECTIVITE (%) EN** | | |
|---|---|---|---|---|
| | | **F143a** | **F125** | **F134a** |
| 31 | 43,1 | 12,9 | 84,6 | 2,5 |
| 50 | 40,9 | 13,0 | 84,2 | 2,8 |
| 75 | 40,0 | 12,8 | 84,9 | 2,3 |
| 80 | 40,4 | 12,4 | 85,4 | 2,2 |
| 143 | 38,2 | 11,1 | 86,6 | 2,3 |
| 177 | 36,9 | 18,0 | 79,8 | 2,2 |
| 180 | 37,6 | 14,2 | 83,8 | 2,0 |
| 193 | 36,6 | 13,0 | 84,8 | 2,2 |
| 218 | 34,4 | 14,6 | 83,0 | 2,4 |
| 241 | 33,8 | 15,7 | 81,8 | 2,5 |
| 260 | 33,0 | 15,0 | 82,5 | 2,5 |
| 270 | 32,6 | 15,7 | 81,9 | 2,4 |
| 290 | 32,1 | 16,0 | 81,7 | 2,3 |
| 330 | 30,0 | 15,6 | 82,0 | 2,4 |
| 350 | 29,8 | 15,2 | 82,3 | 2,5 |
| 390 | 28,3 | 16,0 | 81,6 | 2,4 |
| 415 | 27,4 | 15,0 | 82,7 | 2,3 |
| 430 | 27,1 | 15,7 | 82,0 | 2,3 |
| 450 | 26,1 | 16,0 | 81,7 | 2,3 |
| 500 | 24,0 | 15,6 | 82,1 | 2,3 |

### EXEMPLE 5

Dans le même réacteur qu'à l'exemple 4, on introduit 75 ml du même catalyseur Pd/C commercial à 2 % qu'à l'exemple 4. Préalablement à l'introduction des réactifs, le catalyseur est traité avec de l'hydrogène contenant 100 ppm d'hydrogène sulfuré (H₂S) à température ambiante pendant 60 heures avec un débit gazeux de 6 l/h. Puis il est réduit à 300°C sous pression atmosphérique d'hydrogène. La diffraction des rayons X met en évidence la formation d'une phase Pd₄S.

On fait passer sur le catalyseur un mélange d'hydrogène, de pentafluoroéthane (F125) et de chloropentafluoroéthane (F115) dans les conditions opératoires suivantes :
- Température: : 250°C
- Débit d'hydrogène: : 0,103 mole/heure
- Débit de F125: : 0,281 mole/heure
- Débit de F115: : 0,018 mole/heure

L'analyse faite par chromatographie (CPV) en ligne à la sortie du réacteur donne les résultats rassemblés dans le tableau suivant. On note une activité constante du catalyseur dans le temps.

| **TEMPS (heures)** | **CONVERSION (%) du F115** | **SELECTIVITE (%) EN** | | |
|---|---|---|---|---|
| | | **F143a** | **F125** | **F134a** |
| 10 | 32,4 | 35,2 | 64,8 | 1,0 |
| 21 | 31,8 | 36,8 | 62,2 | 1,0 |
| 30 | 32,8 | 34,9 | 63,7 | 1,4 |
| 40 | 32,5 | 35,1 | 63,8 | 1,1 |
| 72 | 32,0 | 32,6 | 65,9 | 1,5 |
| 140 | 31,3 | 33,3 | 65,5 | 1,2 |
| 150 | 32,3 | 27,3 | 71,3 | 1,4 |
| 165 | 32,0 | 27,5 | 71,2 | 1,3 |
| 189 | 32,0 | 26,5 | 72,0 | 1,5 |
| 250 | 32,1 | 24,8 | 73,7 | 1,5 |
| 280 | 32,6 | 22,0 | 76,5 | 1,5 |
| 290 | 31,8 | 21,5 | 77,0 | 1,5 |
| 306 | 33,0 | 21,1 | 77,4 | 1,5 |
| 350 | 32,3 | 19,5 | 79,0 | 1,5 |
| 395 | 31,8 | 19,0 | 79,5 | 1,5 |
| 430 | 32,4 | 18,1 | 80,4 | 1,5 |
| 450 | 31,5 | 18,0 | 80,5 | 1,5 |
| 500 | 32,2 | 17,6 | 80,9 | 1,5 |

### EXEMPLE 6

### a) Traitement du catalyseur

Dans un évaporateur rotatif, on charge 75 ml du même catalyseur commercial Pd/C à 2 % qu'à l'exemple 4, puis on introduit 100 ml d'une solution d'éthanol contenant 0,007 mole/litre de CS₂. Le solide est maintenu en contact avec la solution à 20°C pendant 20 heures. Le catalyseur est ensuite récupéré par filtration, puis réduit à 300°C sous pression atmosphérique d'hydrogène pendant 4 heures. La quantité de soufre fixé est de 0,15 % en poids et la diffraction des rayons X met en évidence la formation d'une phase Pd₄S.

### b) Synthèse du F125

Dans le même réacteur tubulaire qu'à l'exemple 4, on introduit 75 ml du catalyseur préparé ci-dessus, puis on fait passer dans le réacteur un mélange d'hydrogène et de chloropentafluoroéthane (F115) dans les conditions opératoires suivantes :
- Température: : 250°C
- Débit d'hydrogène: : 0,147 mole/heure
- Débit de F115: : 0,026 mole/heure

Les résultats des analyses faites par chromatographie (CPV) en ligne à la sortie du réacteur sont rassemblés dans le tableau suivant. On note une bonne stabilité de l'activité du catalyseur dans le temps.

| **TEMPS (heures)** | **CONVERSION (%) du F115** | **SELECTIVITE (%) EN** | | |
|---|---|---|---|---|
| | | **F125** | **F143a** | **F134a** |
| 175 | 47,8 | 81,7 | 16,2 | 2,1 |
| 181 | 47,4 | 82,1 | 15,9 | 2,0 |
| 191 | 47,1 | 82,5 | 15,5 | 2,0 |
| 201 | 46,9 | 83,0 | 15,0 | 2,0 |
| 211 | 47,3 | 83,7 | 14,4 | 1,9 |
| 219 | 47,2 | 83,8 | 14,3 | 1,9 |
| 229 | 47,0 | 83,9 | 14,1 | 2,0 |
| 239 | 45,8 | 83,2 | 14,7 | 2,1 |
| 251 | 45,8 | 83,3 | 14,6 | 2,1 |
| 261 | 45,4 | 83,3 | 14,6 | 2,1 |
| 271 | 45,4 | 83,1 | 14,7 | 2,2 |
| 281 | 45,2 | 83,1 | 14,7 | 2,2 |
| 300 | 46,0 | 83,4 | 14,5 | 2,1 |
| 310 | 46,9 | 83,0 | 15,0 | 2,0 |

## Revendications

1. Procédé d'hydrogénolyse en phase gazeuse de chlorofluorocarbures ou d'hydrochlorofluorocarbures en présence d'un catalyseur à base de palladium déposé sur un support, caractérisé en ce que l'on incorpore du soufre au catalyseur.

2. Procédé selon la revendication 1, dans lequel la quantité de soufre par gramme de palladium est comprise entre 0,75 et 750 mg, de préférence entre 2 et 100 mg et plus particulièrement entre 7,5 et 75 mg.

3. Procédé selon la revendication 1 ou 2, dans lequel le palladium représente 0,1 à 10 % du poids total du catalyseur.

4. Procédé selon l'une des revendications 1 à 3, dans lequel le soufre est incorporé au catalyseur au moyen d'un précurseur choisi parmi le chlorure de soufre, le dichlorure de soufre, le disulfure de carbone, le thiophène, l'hydrogène sulfuré, le méthylmercaptan et le diméthylsulfure.

5. Procédé selon l'une des revendications 1 à 4, dans lequel le soufre est incorporé au catalyseur par imprégnation au moyen d'une solution d'un précurseur normalement liquide et traitement sous hydrogène à une température comprise entre 150 et 400°C.

6. Procédé selon la revendication 5, dans lequel on utilise une solution éthanolique de disulfure de carbone.

7. Procédé selon l'une des revendications 1 à 4, dans lequel le soufre est introduit sur le catalyseur par la phase gazeuse avant et/ou pendant la réaction d'hydrogénolyse.

8. Procédé selon la revendication 7, dans lequel le précurseur introduit sous forme gazeuse est l'hydrogène sulfuré, le méthylmercaptan ou le disulfure de carbone.

9. Application du procédé selon l'une des revendications 1 à 8 à l'hydrogénolyse du chloropentafluoroéthane en pentafluoroéthane.

10. Application du procédé selon l'une des revendications 1 à 8 à la purification d'un pentafluoroéthane brut contenant du chloropentafluoroéthane.

## Claims

1. Process for the gas-phase hydrogenolysis of chlorofluorocarbons or of chlorofluorohydrocarbons in the presence of a palladium-based catalyst deposited on a support, characterized in that sulphur is incorporated into the catalyst.

2. Process according to Claim 1, in which the amount of sulphur per gram of palladium is between 0.75 and 750 mg, preferably between 2 and 100 mg and more particularly between 7.5 and 75 mg.

3. Process according to Claim 1 or 2, in which the palladium represents from 0.1 to 10% of the total weight of the catalyst.

4. Process according to one of Claims 1 to 3, in which the sulphur is incorporated into the catalyst using a precursor chosen from sulphur chloride, sulphur dichloride, carbon disulphide, thiophene, hydrogen sulphide, methyl mercaptan and dimethyl sulphide.

5. Process according to one of Claims 1 to 4, in which the sulphur is incorporated into the catalyst by impregnation using a solution of a precursor which is normally liquid, and treatment under hydrogen at a temperature of between 150 and 400°C.

6. Process according to Claim 5, in which an ethanolic solution of carbon disulphide is used.

7. Process according to one of Claims 1 to 4, in which the sulphur is introduced onto the catalyst via the gaseous phase before and/or during the hydrogenolysis reaction.

8. Process according to Claim 7, in which the precursor introduced in gaseous form is hydrogen sulphide, methyl mercaptan or carbon disulphide.

9. Application of the process according to one of Claims 1 to 8, to the hydrogenolysis of chloropentafluoroethane to pentafluoroethane.

10. Application of the process according to one of Claims 1 to 8, to the purification of a crude pentafluoroethane containing chloropentafluoroethane.

## Patentansprüche

1. Verfahren zur Gasphasenhydrogenolyse von Fluorchlorkohlenstoffen oder Fluorchlorkohlenwasserstoffen in Gegenwart eInes Katalysators auf Basis von Palladium, aufgebracht auf einen Träger, dadurch gekennzeichnet, daß man dem Katalysator Schwefel zusetzt.

2. Verfahren nach Anspruch 1, bei dem die Menge an Schwefel pro Gramm Palladium zwischen 0,75 und 750 mg, vorzugsweise zwischen 2 und 100 mg, insbesondere zwischen 7,5 und 75 mg, liegt.

3. Verfahren nach Anspruch 1 oder 2, bei dem das Palladium 0,1 bis 10 % des Gesamtgewichtes des Katalysators darstellt.

4. Verfahren nach einem der Ansprüche 1 bis 3, bei dem der Schwefel dem Katalysator mittels eines Vorläufers zugesetzt wird, der ausgewählt ist aus Chlorschwefel (Dischwefeldichlorid), Schwefeldichlorid, Kohlenstoffdisulfid, Thiophen, Schwefelwasserstoff, Methylmercaptan und Dimethylsulfid.

5. Verfahren nach einem der Ansprüche 1 bis 4, bei dem der Schwefel dem Katalysator zugesetzt wird, indem man ihn mit einer Lösung eines normalerweise flüssigen Vorläufers imprägniert und ihn bei einer Temperatur zwischen 150 und 400 °C unter Wasserstoff behandelt.

6. Verfahren nach Anspruch 5, bei dem man eine ethanolische Lösung von Kohlenstoffdisulfid verwendet.

7. Verfahren nach einem der Ansprüche 1 bis 4, bei dem der Schwefel dem Katalysator vor und/oder während der Hydrogenolysereaktion über die Gasphase zugesetzt wird.

8. Verfahren nach Anspruch 7, bei dem der Vorläufer, der in gasförmiger Form zugegeben wird, Schwefelwasserstoff, Methylmercaptan oder Kohlenstoffdisulfid ist.

9. Anwendung des Verfahrens nach einen, der Ansprüche 1 bis 8 zur Hydrogenolyse von Chlorpentafluorethan zu Pentafluorethan.

10. Anwendung des Verfahrens nach einem der Ansprüche 1 bis 8 zur Reinigung eines Chlorpentafluorethan enthaltenden Rohpentafluorethans.
